# EUROPÄISCHE PATENTSCHRIFT

(11) **EP 1 732 456 B1**
(45) Veröffentlichungstag und Bekanntmachung des Hinweises auf die Patenterteilung: **23.11.2011**
(21) Anmeldenummer: 05716206.7
(22) Anmeldetag: 18.03.2005
(51) Int. Cl.: A61B 17/32

(54) **GERÄT FÜR DIE WASSERSTRAHLCHIRURGIE**
WATER JET SURGERY DEVICE
APPAREIL POUR CHIRURGIE AU JET D'EAU

(30) Priorität: 07.04.2004 DE 102004017261; 29.04.2004 DE 102004021035
(43) Veröffentlichungstag der Anmeldung: 20.12.2006
(73) Patentinhaber: ERBE Elektromedizin GmbH, 72072 Tübingen (DE)
(72) Erfinder: KÜHNER, Ralf, 70567 Stuttgart (DE); HAGG, Martin, 72827 Wannweil (DE); SCHMIDT, Stefanie, 72124 Pliezhausen (DE)
(74) Vertreter: Bohnenberger, Johannes
(86) Internationale Anmeldenummer: PCT/EP2005/002917
(87) Internationale Veröffentlichungsnummer: WO 2005/099595

(56) Entgegenhaltungen:
- WO-A-02/07798
- DE-A1- 19 904 640
- US-A- 5 620 414

## Beschreibung

Die Erfindung betrifft ein Gerät für die Wasserstrahlchirurgie, bei welcher ein Fluid unter hohem Druck zum selektiven Schneiden von Gewebe aus einer Düse ausgestoßen wird.

Üblicherweise wird bei einem solchen Gerät ein Vorratszylinder mit einer (zunächst verschlossenen) Auslassöffnung auf seiner einen Stirnfläche mit einem Arbeitsfluid, z. B. Ringerlösung befüllt und auf seiner anderen Seite mittels eines Kolbens verschlossen. Dieser Vorratszylinder wird dann in eine stabile Kammer in einem Gerätegehäuse eingesetzt. Nach Schließen des Gehäuses fährt ein Hydraulikzylinder eine Betätigungsstange zum Kolben im Vorratszylinder. Nun ist das bekannte Gerät betriebsbereit. Im Betrieb wird der notwendige Arbeitsdruck des Fluids durch den Hydraulikzylinder erzeugt.

Um bei einer Operation eine hinreichende Menge an Arbeitsfluid zur Verfügung zu haben, so dass ein Wechseln des Vorratszplinders mit der damit einhergehenden nicht unbeträchtlichen Unterbrechung der Operation vermieden werden kann, muss der Vorratszylinder relativ großvolumig sein. Je größer man nun die Zylinderbohrung macht, desto größer muss die von der Hydraulik aufgebrachte Kraft und demzufolge auch umso höher die Stabilität der Aufnahme für den Vorratszylindern sein. Wenn andererseits der Hub des Kolbens bzw. die Länge des Vorrats zylinders zur Erhöhung dessen Volumens vergrößert wird, so steigt die Gesamtbauhöhe des Gerätes. Der heutige Kompromiss führt zu Vorratszylindern mit einem Durchmesser von ca. 60 mm und damit zu Geräten, die als Standgeräte ausgebildet sind und eine Bauhöhe von ca. 1.200 bis 1.500 mm aufweisen. Die notwendigen Betätigungseinrichtungen, also der Hydraulikzylinder samt der dazugehörigen Hydraulikpumpe sind äußerst aufwändig und im Betrieb sehr schwerfällig.

Darüber hinaus muss bei "kleinen" Operationen, bei denen nur eine geringe Fluidmenge verbraucht wird, das nichtverwendete Arbeitsfluid aufgrund der hier extrem hohen Anforderungen an die Sterilität des Arbeitsfluids verworfen werden, was die Betriebskosten des bekannten Gerätes erhöht.

Näheres zum Stand der Technik enthält die DE 199 04 640, die ein Gerät für die Wasserstrahlchirurgie gemäß dem Oberbegriff des Anspruchs 1 beschreibt.

WO 02/07798 beschreibt ein System und ein Verfahren zur Gewährleistung eines stetigen Fluidflusses durch eine Hauptleitung. Das System umfasst mindestens zwei Fluidbehälter, von denen jeder eine an die Hauptleitung koppelbare Fluidauslassleitung, eine an die jeweilige Fluidauslassleitung montierte, automatische Schließvorrichtung und einen Controller zur automatischen Feststellung eines Leerens eines der Fluidbehälter und zur automatischen Öffnung einer automatischen Schließvorrichtung bei gefülltem Behälter.

Das US Patent 5,620,414 betrifft eine Vorrichtung und ein Verfahren zur Durchführung eines chirurgischen Schnittes mittels eines Fluidstrahls. Dabei wird sowohl einen hohen Druck als auch Sterilität dadurch erreicht, dass eine kollabierbare Tüte sterilen Wassers von einer Implosionskammer umschlossen wird, die selbst mit einem Fluid gefüllt ist. Die kollabierbare Tüte weist einen abgedichteten Auslass durch eine Öffnung in der Implosionskammer auf. Wenn ein Druck auf das Fluid innerhalb der Implosionskammer mittels eines hydraulischen Kolbens ausgeübt wird, fließt das sterile Wasser aus der kollabierbaren Tüte unter extrem hohen Druck durch ihren Auslass und wird mittels einer handgeführten Schneiddüse an eine Schnittstelle geführt.

Der Erfindung liegt die Aufgabe zu Grunde, ein einfaches Gerät aufzuzeigen, das einen kostengünstigen Betrieb bei verringertem Geräteaufwand ermöglicht.

Diese Aufgabe wird durch das Gerät für die Wasserstrahlchirurgie gemäß Anspruch 1 gelöst. Dabei wird eine Vielzahl von Vorratszylindern vorgesehen, in denen ein Arbeitsfluid gespeichert und mittels eines Kolbens durch einen Auslass in eine Druckleitung ausstoßbar ist. Es ist mindestens eine Betätigungseinrichtung zum Betätigen der Kolben vorgesehen. Weiterhin ist eine Wechseleinrichtung zum Wechseln der Betätigung vom Kolben eines Vorratszylinders zum Kolben eines anderen Vorratszylinders derart vorgesehen, dass das Arbeitsfluid aus nacheinander entleerten Vorratszylindern in die Druckleitung ausstoßbar ist.

Ein wesentlicher Punkt der Erfindung liegt somit darin, dass der bisher vorgesehene eine (einzige) Vorratszylinder aufgeteilt wird in eine Vielzahl von dementsprechend kleineren Vorratszylindern. Durch diese Maßnahme werden mehrere Probleme gleichzeitig gelöst. Zum Ersten können kleinere Vorratszylinder aufgrund ihres geringeren Durchmessers aus schwächer ausgelegtem Material gebaut werden, das dann dennoch den herrschenden (sehr hohen) Drücken standhält. Zum Zweiten kann der notwendige Arbeitsdruck mit einer geringeren Kraft erzeugt werden, so dass die notwendigen Betätigungseinrichtungen einfacher aufgebaut werden können. Zum Dritten kann das Gerät sehr viel kleiner (z. B. als Tischgerät) aufgebaut werden, da dies durch den geringeren Hub der Kolben ermöglicht wird. Zum Vierten werden bei einer "kleineren" Operation weniger Vorratszylinder verbraucht als bei einer "größeren" Operation, so dass die nichtverbrauchten Vorratszylinder für eine nächste Operation zur Verfügung stehen, wobei die Sterilität weiterhin gewährleistet ist.

Vorzugsweise ist die Wechseleinrichtung derart ausgebildet, dass die nacheinander betätigten Kolben überlappend zum unterbrechungsfreien Ausstoß des Fluids in die Druckleitung betätigbar sind. Während einerseits bei den bisher üblichen großvolumigen Vorratszylindern eine solche unterbrechungsfreie Versorgung mit Arbeitsfluid infolge hohen

Fluidvolumens gewährleistet ist, muss gemäß der vorliegenden Erfindung nur ein sehr geringer Aufwand getrieben werden, um diese unterbrechungsfreie Versorgung mit Fluid zu gewährleisten.

Vorzugsweise sind Dichteinrichtungen vorgesehen zum Abdichten des Fluid-Auslasses am Vorratszylinder, so dass keine manuelle, feste Kopplung, sondern lediglich ein kräftiges, selbsttätig/mechanisches Ankoppeln des Vorratszylinders an die Dichteinrichtung ausreicht.

Eine überlappende Betätigung der Kolben ist dann besonders einfach, wenn eine Vielzahl von Betätigungseinrichtungen vorgesehen ist. Ein Wechseln der Betätigungseinrichtungen von einem Kolben zum nächsten Kolben kann dann erspart und durch eine einfache (elektronische) Steuerung ersetzt werden, wenn die Anzahl der Betätigungseinrichtungen der Anzahl der Vorratszylinder im Gerät entspricht. Es ist jedoch auch möglich, mit einer geringeren Anzahl von Betätigungseinrichtungen (im Minimalfall mit einer einzigen Betätigungseinrichtung) zu arbeiten, wenn die Wechseleinrichtung dafür sorgt, dass der Betätigungseinrichtung nacheinander immer wieder neue Vorratszylinder zugeführt werden.

Die Kolben sind vorzugsweise derart mit Rücklaufsperren versehen, dass ein Kolben nach Ausstoß von Arbeitsfluid aus der erreichten Stellung, insbesondere au s seiner Endstellung nach Ausstoßen des gesamten Arbeitsfluids nicht in eine vorherige Stellung zurückschiebbar ist. Auf diese Weise kann gewährleistet werden, dass eine Wiederbefüllung verhindert und ein Arbeiten mit unsterilem Medium sicher unterbunden werden kann.

Am Auslass der Vorratszylinder ist vorzugsweise eine irreversibel öffenbare Transportdichtung vorgesehen. Auch diese Maßnahme bewirkt, dass die Verwendung unsterilem Mediums verhindert werden kann. Darüber hinaus kann eine solche irreversibel öffenbare Transportdichtung sehr einfach ausgebildet und derart gestaltet sein, dass sie beim Einsetzen eines Vorratszylinders oder bei seiner ersten Betätigung selbsttätig geöffnet wird. Aufgrund der hier angewendeten sehr hohen Drücke ist es möglich, die Transportdichtung auch bei erstmaligem Druckaufbau sich selbsttätig öffnend auszubilden. Vorzugsweise wird ein Wechselmagazin vorgesehen, das eine Gruppe von Vorratszylin-dern aufnimmt. Dadurch ergibt sich eine besonders einfache Handhabung sowohl beim Einsetzen von mehreren Vorratszylindern in das Gerät als auch beim Wechseln von einzelnen Betätigungseinrichtungen von Vorratszylinder zu Vorratszylinder. Das Wechselmagazin weist vorzugsweise Kammern auf, welche die Vorratszylinder eng umschließen. Die Vorratszylinder können in diesem Fall mit einer ganz besonders dünnen Wandlung ausgebildet werden, die beim Aufbau des Druckes sich etwas dehnt und dann an den Wänden des Wechselmagazins zur Anlage kommt. Das Behältnis kann auch ausreichend formstabil ausgebildet werden, um dem Druck ohne Formschluß zum Gehäuse standzuhalten.

Im Wechselmagazin sind vorzugsweise Sammeleinrichtungen vorgesehen, zum Zuführen von Arbeitsfluid aus mehreren Vorratszylindern zur Druckleitung. Dies ist besonders dann von Vorteil, wenn ein überlappendes Zuführen von Arbeitsfluid aus verschiedenen Vorratszylindern durchgeführt wird.

Vorzugsweise sind Entlüftungseinrichtungen insbesondere zum gleichzeitigen Entlüften von Leitungsabschnitten zwischen den Auslässen der Vorratszylinder und der Druckleitung vorgesehen, so dass keine Unterbrechung oder kein Pulsieren des Arbeitsfluids beim Entlassen von Luftresten auftritt. Vorzugsweise sind die Entlüftungseinrichtungen im Wechselmagazin angeordnet.

Bei einer Ausführungsform der Erfindung ist das Wechselmagazin mit der Druckleitung fest verbunden und zum einmaligen Gebrauch bestimmt. In diesem Fall kann das Wechselmagazin auch bereits vom Hersteller mit Vorratszylindern befüllt werden, so dass man dann zwar nicht mehr den Vorteil der Ersparnis von Arbeitsflüssigkeit (samt den Vorratszylindern) erhält, dennoch aber alle weiteren oben genannten Vorteile erhalten bleiben. Als besonderer Vorteil kommt hier die leichte Handhabbarkeit insbesondere in Bezug auf Sterilitätskriterien zum Tragen.

Bevorzugte Ausführungsformen der Erfindung ergeben sich aus den Unteransprüchen und den nachfolgenden Ausführungsbeispielen, die anhand von Abbildungen näher erläutert werden. Hierbei zeigen
- - Fig. 1: eine schematische Schnittdarstellung einer ersten Ausführungsform der Erfindung mit einem eingesetzten Magazin vor einer Betätigung,
- - Fig. 2: die Anordnung nach Fig. 1 während der Anfangsphase einer Betätigung eines Zylinders,
- - Fig. 3: die zuvor gezeigte Anordnung während des Ausstoßens von Arbeitsfluid,
- - Fig. 4: die zuvor gezeigte Anordnung mit einem leeren Vorratszylinder und einem weiteren Vorratszylinder während der Betätigung,
- - Fig. 5: eine perspektivische Teil-Darstellung der Anordnung nach den Fig. 1-4,
- - Fig. 6: eine perspektivische Teil-Draufsicht auf eine andere Ausführungsform der Erfindung,
- - Fig. 7: eine Teil-Explosionsdarstellung der Anordnung nach Fig. 6 und
- - Fig. 8: eine schematisierte Darstellung einer Steuerung für die Anordnung nach den Fig. 1 - 5.

In der nachfolgenden Beschreibung werden für gleiche und gleichwirkende Teile dieselben Bezugsziffern verwendet.

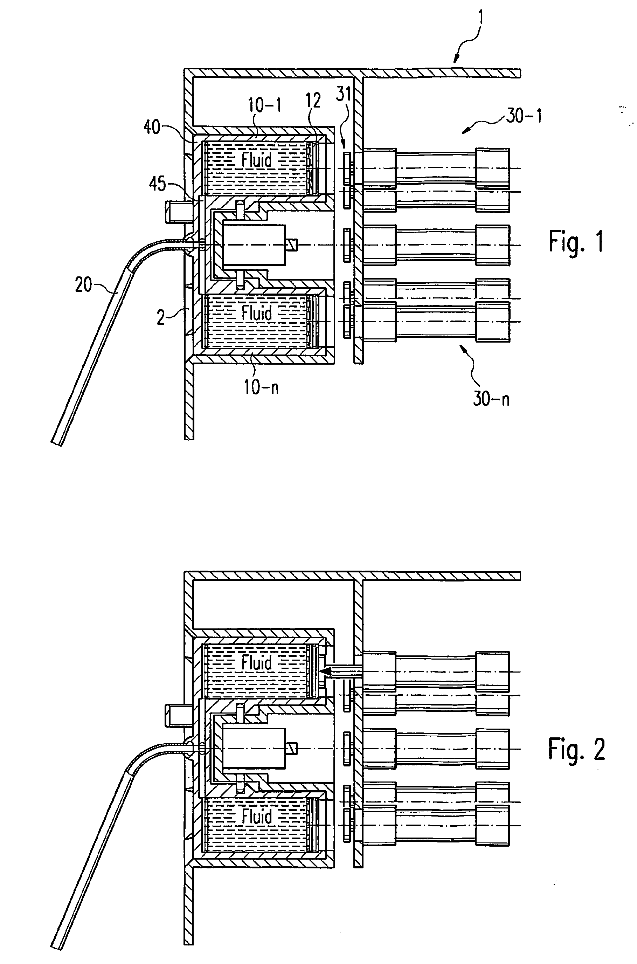

Bei der in den Fig. 1 - 5 dargestellten ersten Ausführungsform der Erfindung ist ein Gerätegehäuse 1 vorgesehen, welches eine frontseitige Öffnung 2 aufweist, in die ein Wechselmagazin 40 eingesetzt werden kann.

Im Wechselmagazin ist eine Vielzahl von Vorratszylindern 10 - 1 bis 10 - n eingesetzt und zwar bei der hier gezeigten Ausführungsform 8 solche Vorratszylinder 10. Aus den Abbildungen geht hervor, dass in diesem Fall die Vorratszylinder 10 direkt im Wechselmagazin 40 ausgebildet oder von diesem gebildet sind und nicht als gesonderte Zylinder eingesetzt werden.

Jeder der Vorratszylinder 10 weist einen Kolben 12 auf, der ihn an einem Ende verschließt. Im Inneren der Vorratszylinder 10 ist Arbeitsfluid, insbesondere Ringerlösung dicht eingeschlossen.

Jeder Vorratszylinder steht mit einem Auslass 13 mit einem Sammelkanal 45 und dieser wiederum mit einer Druckleitung 20 in Verbindung.

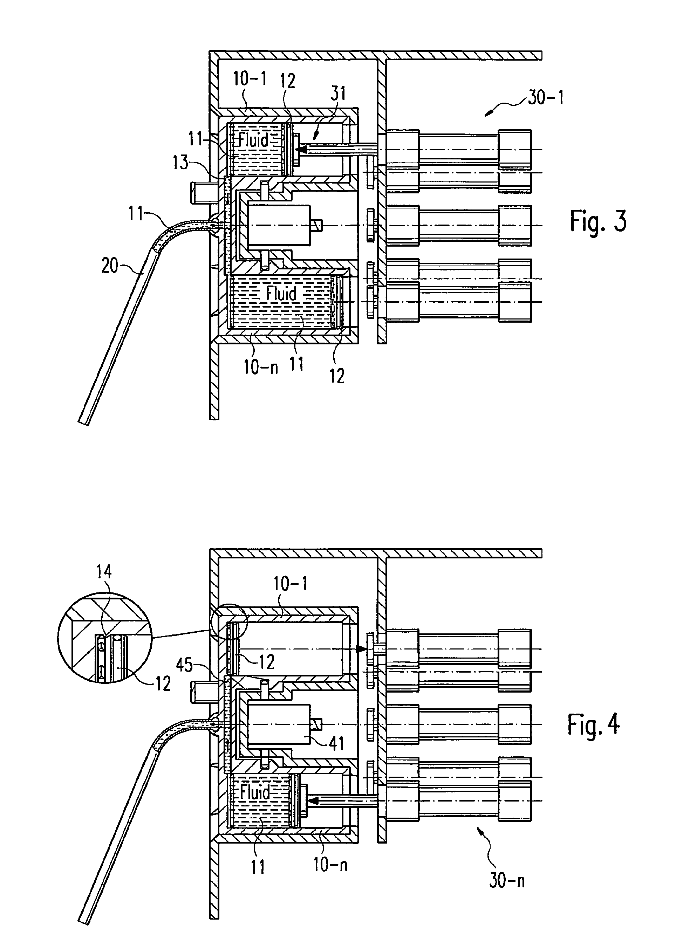

Die Kolben 12 weisen - wie in dem vergrößerten Ausschnitt der Fig. 4 gezeigt - eine Rücklaufsperre 14 auf, die den Kolben 12 in seiner Endposition hält, wenn das gesamte Fluid ausgestoßen ist, wie dies an sich bei Einmal-Spritzen bekannt ist.

Das Wechselmagazin 40 wird nach dem Einsetzen in die Öffnung 2 des Gehäuses 1 an einem Halter 41 verriegelt, so dass es in dem Gerätegehäuse 1 fest gehalten ist.

Im Gehäuse 1 sind weiterhin Betätigungseinrichtungen 30 - 1 bis 30 - n vorgesehen, die hier als Hydraulikzylinder gezeichnet sind, aber natürlich auch als elektromotorisch betriebene Kugelumlaufspindeln oder dergleichen ausgebildet sein können.

Jede der Betätigungseinrichtungen 30 weist einen Stempel 31 auf, der im Gehäuse 1 derart angeordnet ist, dass bei eingesetztem Wechselmagazin 40 die Betätigungsstempel 31 dem ihnen zugewandten Kolben 12 gegenüberliegen.

Die Einsetz-Position, in welcher das Wechselmagazin 40 gerade ins Gehäuse 1 eingesetzt wurde, ist in Fig. 1 gezeigt. In dieser Position sind somit der Sammelkanal 5 und die Druckleitung 20 noch leer.

Wenn nun die erste Betätigungseinrichtung 30 - 1 betätigt und damit ihr Stempel 31 vorgefahren wird, so drückt dieser - wie in Fig. 3 gezeigt - den Kolben 12 in den Vorratszylinder 10 hinein, so dass Fluid 11 durch einen Auslass 13 des ersten Vorratszylinders 10 - 1 in den Sammelkanal 45 und durch diesen in die Druckleitung 20 zum Arbeitsinstrument (nicht gezeigt) gelangt.

Sobald der erste Vorratszylinder 10 - 1 leer ist (siehe Fig. 4), wird ein anderer Vorratszylinder 10 - n durch den Stempel 31 der dazugehörigen Betätigungseinrichtung 30 - n zum Liefern seines gespeicherten Arbeitsfluids 11 "angesteuert". Der Kolben 12 des entleerten Vorratszylinders 10 - 1 wird hierbei in seiner Endstellung durch die Rücklaufsperre 14 gehalten, so dass das aus dem Vorratszylinder 10 - n und den Sammelkanal 45 auf ihn wirkende Fluid diesen Kolben 12 dennoch nicht zurückschieben kann.

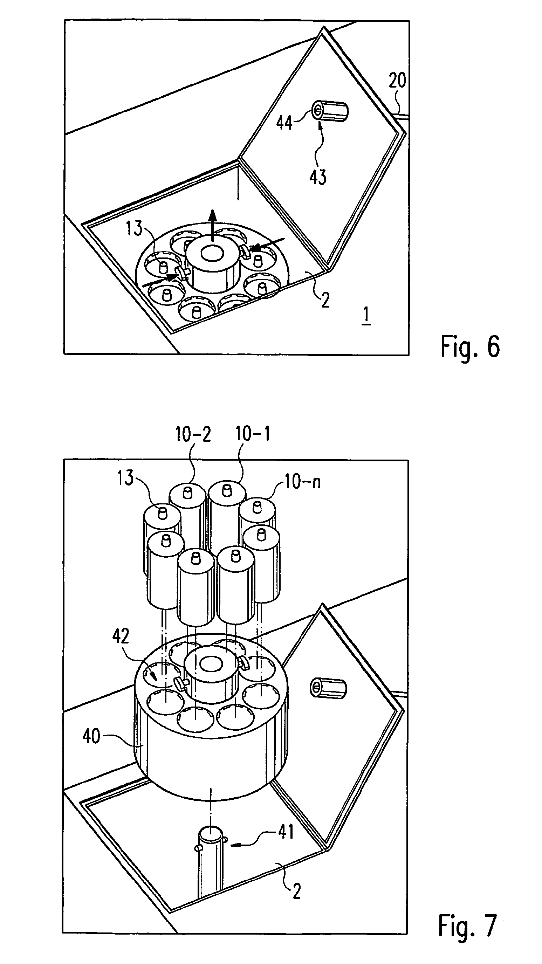

Die in den Fig. 6 und 7 gezeigte Ausführungsform unterscheidet sich von der nach den Fig. 1 - 5 insbesondere dadurch, dass das Wechselmagazin 40 Kammern 42 aufweist, in welche einzelne Vorratszylinder 10 als gesonderte "Bauteile" eingesetzt werden können. Das Wechselmagazin 40 kann bei dieser Anordnung um seine Achse gedreht werden, so dass nacheinander Vorratszylinder 10 - 1 bis 10 - n mit ihrem Auslass 13 in Verbindung mit einer Dichteinrichtung 43 gebracht werden können, über welche eine dichte Verbindung des jeweiligen Vorratszylinders 10 mit der Druckleitung 20 geschaffen werden kann. Zum Öffnen von (nicht gezeigten) Verschlusseinrichtungen auf den Auslässen 13 der Vorratszylinder 10 ist hier eine Öffnungsnadel 44 gezeigt, die dann den Verschluss des jeweiligen Vorratszylinders 10 öffnet, wenn die Dichteinrichtung 43 auf den Auslass 13 des Vorratszylinders 10 aufgedrückt wird. Das Wechseln der Vorratszylinder 10 im Wechselmagazin 40 durch dessen Drehung entspricht somit ein wenig dem Wechseln der Patronen im (bzw. vor dem) Lauf eines Trommelrevolvers.

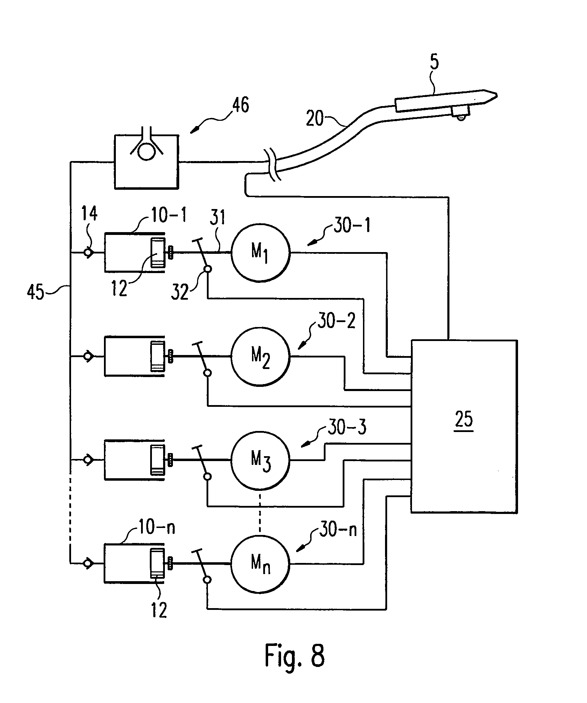

In Fig. 8 ist - sehr stark schematisiert - eine Steuerung für das Gerät nach den Fig. 1 - 5 gezeigt.

Diese Steuerung umfasst einen Rechner 25, der in steuernder Verbindung mit den Betätigungseinrichtungen 30 - 1 bis 30 - n steht. Die Stempel 31 der Betätigungseinrichtungen 30 - 1 bis 30 - n, die im vorliegenden Fall als elektromotorische Antriebe (z. B. mit Kugelumlaufspindeln) ausgebildet sind, drücken auf die Kolben 12 der Vorratszylinder 10. Die Kraft, mit der dies geschieht, kann bei dieser Ausführungsform durch den Strom gesteuert werden, mit welchem die Elektromotoren M₁ - Mₙ durch den Rechner 25 angesteuert werden.

Die Auslässe der Vorratszylinder 10 stehen mit dem Sammelkanal 45 in Verbindung. Zwischen den verschiedenen Vorratszylindern 10 und dem Sammelkanal 45 sind die hier als Rückschlagventile ausgebildeten Rücklaufsperren 14 derart vorgesehen, dass der Druck im Sammelkanal 45 in keinem Fall auf einen Kolben 12 zurückwirken kann, der im Moment nicht mit der entsprechenden Kraft betätigt wird.

Zwischen dem Sammelkanal 45 und der zu einem Arbeitsinstrument 5 führenden Druckleitung 20 ist eine Entlüftungseinrichtung 46 vorgesehen.

Weiterhin sind Weg-Sensoren 32 vorgesehen, welche dem Rechner 25 zumindest dann ein Signal geben, wenn der zugehörige Stempel 31 seine Endposition erreicht hat.

Mit der in Fig. 8 gezeigten Anordnung ist es nun möglich, die Betätigungseinrichtungen 30 - 1 bis 30 - n derart anzusteuern, dass die Vorratszylinder nacheinander und zwar auch überlappend entleert werden können, so dass der Operateur nicht durch Druckschwankungen des Arbeitsfluids gestört wird.

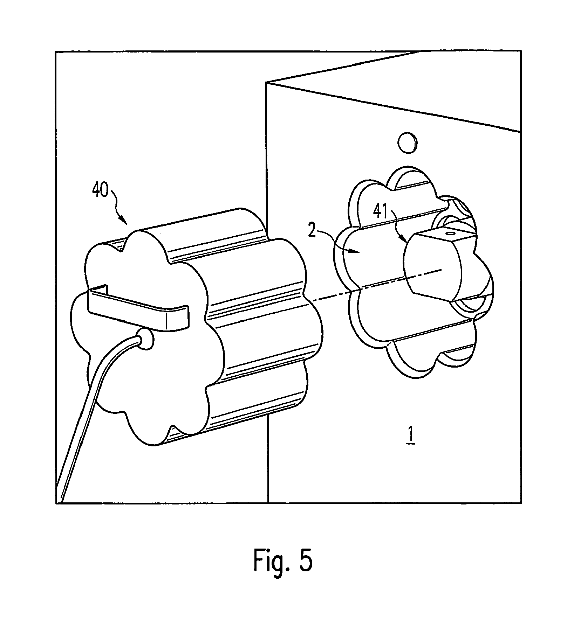

Aus Obigem geht hervor, dass eine Kombination der hier erläuterten Einzelmerkmale ohne Weiteres möglich ist. So kann beispielsweise das in den Fig. 6 und 7 gezeigte Magazin auch durch ein solches nach Fig. 5 (zum einmaligen Gebrauch) ersetzt werden. Die Steuerung durch den hier gezeigten Computer 25 kann natürlich durch eine entsprechende Kurvenscheibenmechanik ersetzt werden.

### Bezugszeichenliste

- 1: Gerätegehäuse
- 2: Öffnung
- 5: Arbeitsinstrument
- 10: Vorratszylinder
- 11: Fluid
- 12: Kolben
- 13: Auslass
- 14: Rücklaufsperre
- 20: Druckleitung
- 25: Rechner
- 30: Betätigungseinrichtung
- 31: Stempel
- 32: Weg-Sensor
- 40: Wechselmagazin
- 41: Halter
- 42: Kammer
- 43: Dichteinrichtung
- 44: Öffnungsnadel
- 45: Sammelkanal
- 46: Entlüftungseinrichtung
- 50: Wechseleinrichtung

## Patentansprüche

1. Gerät für die Wasserstrahlchirurgie, umfassend
eine Vielzahl von Vorratszylindern (10), in denen ein Arbeitsfluid (11) gespeichert und mittels eines Kolbens (12) durch einen Auslass (13) in eine Druckleitung (20) ausstoßbar ist,
mindestens eine Betätigungseinrichtung (30) zum Betätigen des Kolbens (12), eine Wechseleinrichtung (50) zum Wechseln der Betätigung vom Kolben (12) eines Vorratszylinders (10 - 1) zum Kolben (12) eines anderen Vorratszylinders (10 - n) derart, dass das Arbeitsfluid (11) aus nacheinander entleerten Vorratszylindern (10) in die Druckleitung (20) ausstoßbar ist, **gekennzeichnet durch**
ein Wechselmagazin (40) zur Aufnahme einer Gruppe von Vorratszylindern (10).

2. Gerät nach Anspruch 1,
**dadurch gekennzeichnet, dass**
die Wechseleinrichtung (50) derart ausgebildet ist, dass die nacheinander betätigten Kolben (12) überlappend zum unterbrechungsfreien Ausstoß des Fluids (11) in die Druckleitung (20) betätigbar sind.

3. Gerät nach einem der vorhergehenden Ansprüche,
**gekennzeichnet durch**
eine Dichteinrichtung (43) zum Abdichten des Fluidauslasses (13).

4. Gerät nach einem der vorhergehenden Ansprüche,
**dadurch gekennzeichnet, dass**
eine Vielzahl von Betätigungseinrichtungen (30 - 1) vorgesehen ist.

5. Gerät nach einem der vorhergehenden Ansprüche,
**dadurch gekennzeichnet, dass**
die Kolben (12) Rücklaufsperren (14) derart umfassen, dass ein Kolben (12) nach Ausstoß von Arbeitsfluid (11) aus der erreichten Stellung nicht in eine vorherige Stellung zurückschiebbar ist.

6. Gerät nach einem der vorhergehenden Ansprüche,
**dadurch gekennzeichnet, dass**
am Auslass (13) eine vorzugsweise irreversibel öffenbare Transportdichtung vorgesehen ist.

7. Gerät nach einem der vorhergehenden Ansprüche,
**dadurch gekennzeichnet, dass**
das Wechselmagazin (40) Kammern (42) aufweist, welche die Vorratszylinder (10) eng umschließen.

8. Gerät nach einem der Ansprüche 6 oder 7,
**dadurch gekennzeichnet, dass**
im Wechselmagazin (40) Sammeleinrichtungen (45) zum Zuführen von Arbeitsfluid (11) aus mehreren Vorratszylindern (10) zur Druckleitung (20) vorgesehen sind.

9. Gerät nach einem der vorhergehenden Ansprüche,
**gekennzeichnet durch**
Entlüftungseinrichtungen (46) zum vorzugsweise gleichzeitigen Entlüften von Leitungsabschnitten zwischen den Auslässen (13) der Vorratszylinder (10) und der Druckleitung (20).

10. Gerät nach Anspruch 9,
**dadurch gekennzeichnet, dass**
die Entlüftungseinrichtungen in einem Wechselmagazin (40) angeordnet sind.

11. Gerät nach einem der vorhergehenden Ansprüche,
**dadurch gekennzeichnet, dass**
das Wechselmagazin (40) mit der Druckleitung (20) zur Bildung einer Einmal-Einheit irreversibel verbunden ist.

## Claims

1. Tool for water-jet surgery, comprising
a multiplicity of supply cylinders (10), in which an operating fluid (11) is stored and can be discharged by means of a piston (12) through an outlet (13) into a pressure line (20),
at least one operating device (30) for operating the pistons (12),
and a changeover device (50) for changing the operation from the piston of one supply cylinder (10-1) to the piston (12) of another supply cylinder (10-n) in such a way that the operating fluid (11) can be discharged into the pressure line (20) from consecutively emptied supply cylinders (10),
**characterized by**
a changeover magazine (40) for accommodating a group of supply cylinders (10).

2. Tool according to claim 1,
**characterized in that**
the changeover device (50) is designed in such a way that the consecutively operated pistons (12) can be operated in an overlapping manner for the interruption-free discharge of the fluid (11) into the pressure line (20).

3. Tool according to one of the preceding claims,
**characterized by**
a sealing device (43) for sealing the fluid outlet (13).

4. Tool according to one of the preceding claims,
**characterized in that**
provision is made for a multiplicity of operating devices (30-1).

5. Tool according to one of the preceding claims,
**characterized in that**
the pistons (12) include non-return devices (14) in such a way that a piston (12), after discharging operating fluid (11) from the position which is reached, cannot slide back into a previous position.

6. Tool according to one of the preceding claims,
**characterized in that**
a preferably irreversibly openable transport seal is provided at the outlet (13).

7. Tool according to one of the preceding claims,
**characterized in that**
the changeover magazine (40) has chambers (42) which closely encompass the supply cylinders (10).

8. Tool according to either of claims 6 and 7,
**characterized in that**
collecting devices (45), for feeding operating fluid (11) from a plurality of supply cylinders (10) to the pressure line (20), are provided in the changeover magazine (40).

9. Tool according to one of the preceding claims,
**characterized by**
ventilation devices (46) for the preferably simultaneous ventilating of line sections between the outlets (13) of the supply cylinders (10) and the pressure line (20).

10. Tool according to claim 9,
**characterized in that**
the ventilation devices are arranged in a changeover magazine (40).

11. Tool according to one of the preceding claims,
**characterized in that**
the changeover magazine (40) is irreversibly connected to the pressure line (20) for forming a once-through unit.

## Revendications

1. Appareil pour la chirurgie à jet d'eau, comprenant une pluralité de cylindres de réserve (10), dans lesquels un fluide de travail (11) est stocké et peut être expulsé au moyen d'un piston (12) par une sortie (13) dans une conduite sous pression (20),
au moins un dispositif d'actionnement (30) pour l'actionnement du piston (12), un dispositif de remplacement (50) pour le remplacement de la commande depuis le piston (12) d'un cylindre de réserve (10-1) vers le piston (12) d'un autre cylindre de réserve (10-n), de telle sorte que le fluide de travail (11) peut être expulsé de cylindres de réserve (10) vidés les uns après les autres dans la conduite sous pression (20),
**caractérisé par**
un chargeur de remplacement (40) pour la réception d'un groupe de cylindres de réserve (10).

2. Appareil selon la revendication 1,
**caractérisé en ce que**
le dispositif de remplacement (50) est conçu de telle sorte que les pistons (12) actionnés successivement peuvent être actionnés en se chevauchant pour l'éjection sans interruption du fluide (11) dans la conduite sous pression (20).

3. Appareil selon l'une des revendications précédentes,
**caractérisé par**
un dispositif d'étanchéité (43) pour l'étanchéité de la sortie de fluide (13).

4. Appareil selon l'une des revendications précédentes,
**caractérisé en ce qu'**une pluralité de dispositifs d'actionnement (30-1) est prévue.

5. Appareil selon l'une des revendications précédentes,
**caractérisé en ce que**
les pistons (12) comprennent des blocages de retour (14) de telle sorte qu'un piston (12) ne peut pas être reculé, après l'expulsion du fluide de travail (11) de la position atteinte dans une position antérieure.

6. Appareil selon l'une des revendications précédentes,
**caractérisé en ce que**
un joint de transport pouvant être ouvert de préférence de façon irréversible est prévu sur la sortie (13).

7. Appareil selon l'une des revendications précédentes,
**caractérisé en ce que**
le chargeur de remplacement (40) présente des chambres (42) qui entourent de façon ajustée les cylindres de réserve (10).

8. Appareil selon l'une des revendications 6 ou 7, **caractérisé en ce que**
des dispositifs collecteurs (45) pour l'arrivée de fluide de travail (11) comprenant plusieurs cylindres de réserve (10) pour la conduite sous pression (20) sont prévus dans le chargeur de remplacement (40).

9. Appareil selon l'une des revendications précédentes,
**caractérisé par**
des dispositifs de purge (46) pour la purge de préférence simultanée de tronçons de conduite entre les sorties (13) des cylindres de réserve (10) et la conduite sous pression (20).

10. Appareil selon la revendication 9,
**caractérisé en ce que**
les dispositifs de purge sont disposés dans un chargeur de remplacement (40).

11. Appareil selon l'une des revendications précédentes,
**caractérisé en ce que**
le chargeur de remplacement (40) est relié de façon irréversible à la conduite sous pression (20) pour former une unité unique.
